Europäisches Patentamt

(19) European Patent Office

Office européen des brevets

(11) Publication number: **0 015 247**
**B1**

# (12) EUROPEAN PATENT SPECIFICATION

(45) Date of publication of patent specification: 22.12.82

(51) Int. Cl.³: **A 61 M 15/00**

(21) Application number: **80850006.0**

(22) Date of filing: **18.01.80**

(54) An aerosol inhalation device.

(30) Priority: 16.02.79 SE 7901417

(43) Date of publication of application:
03.09.80 Bulletin 80/18

(45) Publication of the grant of the patent:
22.12.82 Bulletin 82/51

(84) Designated Contracting States:
AT BE CH DE FR GB IT LU NL SE

(56) References cited:
GB - A - 1 017 032
US - A - 3 739 950
US - A - 3 994 421

(73) Proprietor: Aktiebolaget DRACO
Fack
S-221 01 Lund 1 (SE)

(72) Inventor: Monö, Rune Gotthard
Näsbyvägen 3
S-183 30 Täby (CH)
Inventor: Morén, Nils Folke Emanuel
Caritasgaten 20 B
S-216 18 Malmö (SE)
Inventor: Wetterlin, Kjell Ingvar Leopold
Västervang 19
S-240 17 S Sandby (SE)

(74) Representative: Wurm, Bengt Runio et al,
Patent and Trade Mark Department Ab Astra
S-151 85 Södertälje (SE)

Courier Press, Leamington Spa, England.

## An aerosol inhalation device

Technical Field

The present invention is related to a device allowing an improved and simplified inhalation of aerosols, especially pharmaceuticals in aerosol form.

Background Art

Therapeutically active substances may be administered by inhalation to the lungs of a patient for producing a local effect therein. Thereby a rapid onset of the effect is obtained using a low dosage of the substance, and systemic side-effects are reduced. Pressurized containers, in which the active substance is dissolved or suspended in a propellant such as a chlorofluorocarbon, are often used.

From such a container a dosage is released via a valve system and inhaled through a mouthpiece. On release of the dosage the particles formed have a high velocity. Furthermore the aerosol droplets generated in the orifice of the mouthpiece are large as they contain residual amounts of propellant. The aerosol droplets will then to a great extent be deposited on the mucous membranes of the oral cavity instead of being inhaled into the pulmonary system. Undesirable local side effects in the oral cavity may then arise.

Furthermore, inhalation of drugs directly from the mouthpiece of a pressurized container requires a coordination between release of the dosage from the container and inspiration by the patient. Some patients have difficulties in performing such coordination.

In order to alleviate these problems it has been proposed to provide a pressurized aerosol container with a cylindrical or pear shaped inhalation tube, for use as a deceleration chamber for the aerosol droplets (Morén, F.; Int. J. Pharm. 1 (1978) 205—212). Such inhalation tubes may be used to avoid to some extent problems referred to above. However, to be an effective aid to the patient in need of inhalation therapy an inhalation device must be easy to put into operation. Devices in which parts must be assembled before use should be avoided in view of the acute conditions, e.g. asthmatic conditions under which some patients must take their medication. While providing a sufficient space for retardation of the drug particles and for evaporation of the propellant, the inhalation device must be sufficiently small to be carried with the patient.

GB—A—1,017,032 and US—A—3,994,421 disclose aerosol inhalation devices wherein the deceleration chamber comprises two telescoping cylindrical tubes, so that the size of the deceleration chamber can be diminished when stored in e.g. a pocket. However, the interior of these devices must be protected from contamination with dust during storage by applying a separate cap or similar on the outlet opening. Such a cap complicates the assembling of the device before use and the cap can also easily be lost.

The object of the present invention is to provide an inhalation device which does not possess the above discussed disadvantages with the known devices.

Disclosure of Invention

The present invention provides an aerosol inhalation device comprising an elongated container for use as a deceleration chamber, provided at a rear end with means for connection thereof to an aerosol dispenser and having an outlet opening at the opposite front end, which outlet opening is arranged in use to be brought to the mouth of a patient, the container comprising two container parts of which an outer container part comprises the outlet opening and is telescopically displaceable over an inner container part, characterised in the two container parts having a substantially rectangular cross-section and in the front end of the inner container part being provided with a flexible tongue, which on telescoping the container together will close the outlet opening.

According to a preferred embodiment of the invention the portion of the outer container part comprising the outlet opening is shaped as a curved end wall along the inside of which the tongue of the inner part is arranged to run. With this embodiment of the invention it is further possible to design the device in such manner that the outlet opening may be tightly connected to the patient's mouth without requiring the patient's lips to be closed around a projecting mouthpiece as being required in prior art inhalation tubes. A further advantage of the device of the invention is that it may be made up of two parts only.

. The invention is further described with reference to the enclosed drawings, of which

Fig. 1 is a longitudinal sectional view of an aerosol inhalation device according to a preferred embodiment of the invention, connected to an aerosol dispenser,

Fig. 2 is a cross sectional view along the line 2—2 of fig. 1,

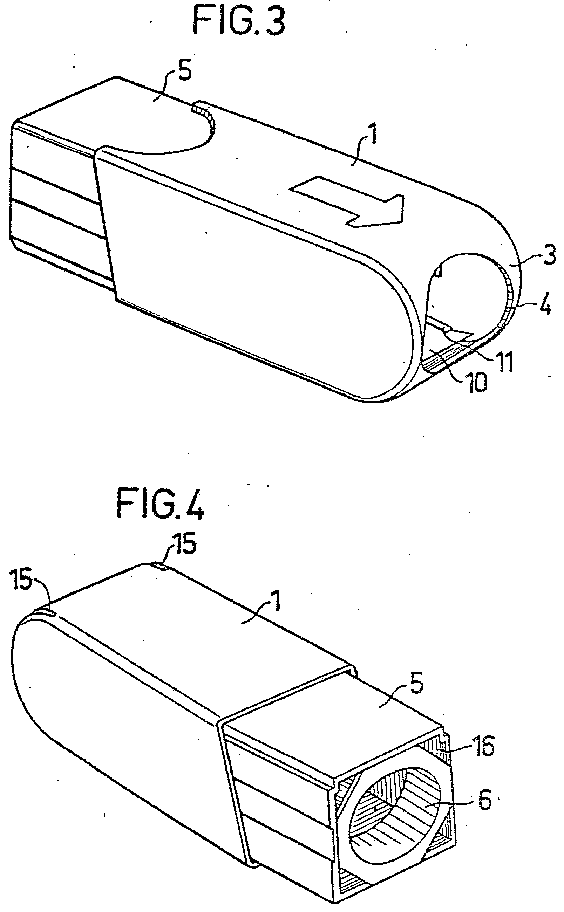

Fig. 3 is a perspective view of the inhalation device shown in fig. 1 as viewed from above, and

Fig. 4 is a perspective view of said device viewed from below.

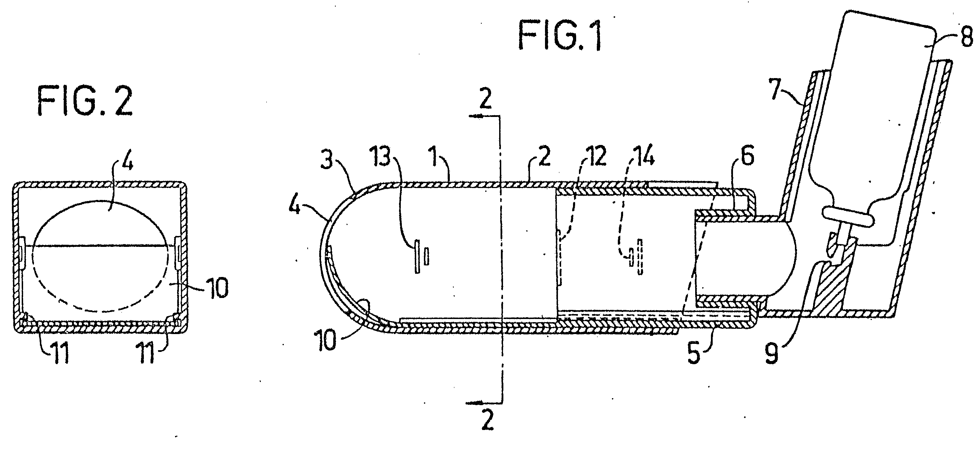

In the drawings 1 represents an outer container part having a portion 2 with a rectangular cross section and having a curved wall 3 with an outlet opening 4 at the front end thereof. The outer container part is telescopically displaceable over an inner container part 5 having such rectangular cross section as to fit within the outer container part and being provided at its rear end with an inwardly oriented sleeve 6 for connection of the inhalation device to a known aerosol dispenser 7 comprising a pressurized container 8 with a metering valve and a spray orifice 9. A flexible tongue 10 is projecting from one of the front edges of the inner container part 5, the flexible tongue being integral with said container part, the tongue being guided along the bottom of the outer container part 1 by guiding means 11 arranged at the side walls thereof. On sliding the two container parts together the free end of the tongue 10 will run along the inside of the curved wall 3, thereby closing the opening 4. The device may be held in a closed (storage) position by means of a locking edge 12 on the outside of the inner container part fitting with edges 13 on the inside of the outer container part. In the open (operating) position the edge 12 will fit with corresponding edges 14. In fig. 1 and 2 the device is shown in an intermediary position between the storage position and the operating position. To further secure against pulling the parts 1 and 5 apart the tongue 10 may be provided with shoulders near the front end thereof engaging in the operating condition of the device with openings 15 in the outer chamber part. At the rear end the device is provided with air inlet openings 16.

The device of the invention may be carried by the patient in the closed (storage) position assembled with an aerosol dispenser 7 comprising a pressurized container 8. The device may then easily be put into operating condition by pulling out the outer container part, whereafter the outlet opening is brought to the patient's mouth, an aerosol dose is released and inhaled through the device. In the known aerosol dispenser shown the dose is released by pressing down the pressurized container.

The device of the invention may be modified within the scope of the appended claims, thus the device may be designed to be connected to an aerosol dispenser different from that shown in figs. 1 and 2. The flexible tongue may be projecting from another front edge of the inner container part.

The length of the inhalation device in the operating position must be more than 5 cm and is suitably 10—20 cm measured from the orifice of the mouthpiece. The device is preferably made of plastic material e.g. polyethylene by injection moulding by the separate parts thereof.

Best Mode of Carrying Out the Invention The best mode of carrying out the invention known at present is the embodiment shown by the drawings and described with reference thereto, the length of the device in operating position being about 13 cm and the device being made of polyethylene.

Claims

1. An aerosol inhalation device comprising an elongated container for use as a deceleration chamber, provided at a rear end with means (6) for connection thereof to an aerosol dispenser (7) and having an outlet opening (4) at the opposite front end, which outlet opening is arranged in use to be brought to the mouth of a patient, the container comprising two container parts (1, 5) of which an outer container part (1) comprises the outlet opening (4) and is telescopically displaceable over an inner container part (5), characterised in the two container parts having a substantially rectangular cross-section and in the front end of the inner container part (5) being provided with a flexible tongue (10), which on telescoping the container together will close the outlet opening.

2. An aerosol inhalation device according to claim 1, characterized in that the portion of the outer container part (1) comprising the outlet opening is shaped as a curved end wall (3) along the inside of which the tongue (10) of the inner part (5) is arranged to run.

3. An aerosol inhalation device according to claims 1 or 2, characterized in that the outside of the inner container part (5) is provided with a locking edge (12) fitting with edges (13, 14) on the inside of the outer container part (1) for locking the device in the closed or open position.

4. An aerosol inhalation device according to one or more of claims 1, 2 and 3, characterized in that the rear end of the inner container part (5) is provided with air inlet openings (16).

Revendications

1. Dispositif d'inhalation d'aérosol comprenant un récipient allongé utilisable comme une chambre de décélération, pourvu à une extrémité arrière de moyens (6) permettant son raccordement avec un distributeur d'aérosol (7) et comportant un orifice de sortie (4) à l'extrémité avant opposée, ledit orifice de sortie étant agencé en service pour être placé sur la bouche d'un patient, le récipient comprenant deux parties (1, 5) dont la partie extérieure (1) comprend l'orifice de sortie (4) et est déplaçable télescopiquement sur une partie intérieure de récipient (5), caractérisé en ce que les deux parties du récipient ont une section droite essentiellement rectangulaire et en ce qu'il est prévu dans l'extrémité avant de la partie intérieure du récipient (5) une languette flexible (10) qui, lors de la compression télescopique du récipient, ferme l'orifice de sortie.

2. Dispositif d'inhalation d'aérosol selon la revendication 1, caractérisé en ce que la portion de la partie extérieure de récipient (1) comprenant l'orifice de sortie est profilée sous la forme d'une paroi extrême incurvée (3) le long de l'intérieur de laquelle est disposée la languette (10) de la partie intérieure (5) de façon à se déplacer.

3. Dispositif d'inhalation d'aérosol selon l'une des revendications 1 ou 2, caractérisé en ce que le côté extérieur de la partie intérieure de récipient (5) est pourvu d'un bord de verrouillage (12) s'accrochant sur les bords (13, 14) prévus sur le côté intérieur de la partie extérieure de récipient (1) pour verrouiller le dispositif dans la position de fermeture ou d'ouverture.

4. Dispositif d'inhalation d'aérosol selon l'une quelconque des revendications 1 à 3, caractérisé en ce que l'extrémité arrière de la partie intérieure de récipient (5) est pourvue d'orifices d'entrée d'air (16).

## Patentansprüche

1. Aerosolinhalationsvorrichtung mit einem länglichen Behälter für die Benutzung als Verzögerungskammer, die am hinteren Ende mit Einrichtungen (6) versehen ist für ihre Verbindung mit einer Aerosolabgabeeinrichtung (7), und mit einer Auslaßöffnung (4) an dem gegenüberliegenden vorderen Ende, wobei die Auslaßöffnung derart angeordnet ist, daß sie bei der Benutzung an den Mund eine Patienten bringbar ist, und der Behälter zwei Behälterteile (1, 5) aufweist, deren äußerer Behälterteil (1) die Auslaßöffnung (4) aufweist und über einen inneren Behälterteil (5) zusammenschiebbar ist, dadurch gekennzeichnet, daß die zwei Behälterteile einen im wesentlichen rechteckigen Querschnitt haben und im vorderen Ende des inneren Behälterteils (5) mit einer flexiblen Zunge (10) versehen sind, die beim Zusammenschieben des Behälters die Auslaßöffnung verschließt.

2. Aerosolinhalationsvorrichtung nach Anspruch 1, dadurch gekennzeichnet, daß das Teil des äußeren Behälterteils (1), welches die Auslaßöffnung aufweist, als eine gekrümmte Endwand (3) ausgestaltet ist, längs deren Innenseite die Zunge (10) des inneren Teils (5) zu laufen angeordnet ist.

3. Aerosolinhalationsvorrichtung nach Anspruch 1 oder 2, dadurch gekennzeichnet, daß die Außenseite des inneren Behälterteils (5) mit einer Sperrkante (12) versehen ist, die mit Kanten (13, 14) auf der Innenseite des äußeren Behälterteils (1) für das Festhalten der Vorrichtung in der geschlossenen oder der offenen Stellung zusammenpaßt.

4. Aerosolinhalationsvorrichtung nach einem oder mehreren der Ansprüche 1, 2 und 3, dadurch gekennzeichnet, daß das hintere Ende des inneren Behälterteils (5) mit Lufteinlaßöffnungen (16) versehen ist.

FIG.2

FIG.1

0015 247

# FIG.3

# FIG.4